# EUROPÄISCHE PATENTANMELDUNG

(11) **EP 2 264 184 A1**
(43) Veröffentlichungstag der Anmeldung: **22.12.2010**
(21) Anmeldenummer: 09163338.8
(22) Anmeldetag: 22.06.2009
(51) Int. Cl.: C12Q 1/68, C12N 15/00

(54) **Verfahren zur Amplifikation von DNA unter Verwendung von Tetraethylenglykol, Kit-of-parts dafür und dessen Verwendung**

(71) Anmelder: Qiagen GmbH, 40724 Hilden (DE)
(72) Erfinder: Attig, Hans, 40724 Hilden (DE); Himmelreich, Ralf, 49724 Hilden (DE); Wende, Andy, 40724 Hilden (DE)
(74) Vertreter: Gille Hrabal Struck Neidlein Prop Roos

(57) **Zusammenfassung**

Offenbart wird eine Verfahren zur Amplifikation von DNA, ein Verfahren zur Präparation und Amplifikation von DNA, ein Kit-of-parts zur DNA-Amplifikation und DNA-Präparation und dessen Verwendung, die alle durch die Verwendung von Tetraethylenglykol gekennzeichnet sind. Weiterhin wird die Verwendung von Tetraethylenglykol in einer Reaktionslösung durch Durchführung einer DNA-Amplifikation und eine Tetraethylenglykol enthaltende Reaktionslösung zur Durchführung einer DNA-Amplifikation offenbart.

SEQUENCE LISTING

<110>  Qiagen GmbH

<120>  Verfahren zur Amplifikation von DNA unter Verwendung von
       Tetraethylenglykol, Kit-of-parts dafür und dessen Verwendung

<130>  H 64 569

<160>  11

<170>  PatentIn version 3.3

<210>  1
<211>  24
<212>  DNA
<213>  artificial sequence

<220>
<223>  Forward Primer

<400>  1
cgatgatgct acccctgaaa aact                                              24

<210>  2
<211>  25
<212>  DNA
<213>  artificial sequence

<220>
<223>  Reverse Primer

<400>  2
tattgtcgct tgaactgatt tcctc                                             25

<210>  3
<211>  25
<212>  DNA
<213>  artificial sequence

<220>
<223>  Sonde

<400>  3
cgttgttaag tcaatggaaa acctg                                             25

<210>  4
<211>  22
<212>  DNA
<213>  artificial sequence

<220>
<223>  Forward Primer

<400>  4
cacacagcga tggcagctat gc                                                22

<210>  5
<211>  19
<212>  DNA
<213>  artificial sequence

<220>
<223>  Reverse Primer

<400>  5
cccagtgatg ggccagctc                                                    19


<210>  6
<211>  23
<212>  DNA
<213>  artificial sequence

<220>
<223>  Forward Primer

<400>  6
cttctgatcc gcatagtgac gac                                               23


<210>  7
<211>  22
<212>  DNA
<213>  artificial sequence

<220>
<223>  Reverse Primer

<400>  7
aacggtcatt cgcctccagc ag                                                22


<210>  8
<211>  23
<212>  DNA
<213>  artificial sequence

<220>
<223>  Sonde

<400>  8
taggggatgg taacgacgaa gca                                               23


<210>  9
<211>  22
<212>  DNA
<213>  artificial sequence

<220>
<223>  Forward Primer

<400>  9
gccgctagag gtgaaattct tg                                                22


<210>  10
<211>  21
<212>  DNA
<213>  artificial sequence

<220>
<223>  Reverse Primer

<400>  10
cattcttggc aaatgctttc g                                                 21


<210>  11
<211>  21
<212>  DNA

<213>  artificial sequence

<220>
<223>  Sonde

<400>  11
accggcgcaa gacggaccag a                                                21

## Beschreibung

Die Erfindung betrifft ein Verfahren zur Amplifikation von DNA bzw. ein Verfahren zur Präparation und Amplifikation von DNA, ein Kit-of-parts zur DNA-Amplifikation, dessen Verwendung sowie eine Reaktionslösung zur Durchführung einer DNA-Amplifikation und die Verwendung von Tetraethylenglykol in einer Reaktionslösung zur Durchführung einer DNA-Amplifikation.

### Hintergrund

Es ist zunehmend von großer Bedeutung, Biopartikel wie Bakterien, Viren, Pilze, Protozoen oder ähnliche in Proben unterschiedlicher Herkunft nachzuweisen.

Eine Möglichkeit dafür besteht darin, die Nukleinsäuren der in einer Probe enthaltenen Zellen freizusetzen, diese anschließend spezifisch zu amplifizieren und dann nachzuweisen. Hierzu sind folgende Schritte erforderlich:
- Materialentnahme (z.B. Blut, Sputum, Urin, Faeces, Liquor, sowie Abstrichproben aus Mundhöhle, Nasenraum, Genitaltrakt; aber auch forensische Proben, Bodenproben etc.)
- Lyse (Aufschließen der Zellen)
- Nukleinsäure-Präparation
- Nukleinsäure-Amplifikation
- Detektion der amplifizierten Nukleinsäuren

Dieses Verfahren ist grundsätzlich bekannt (z.B. Bowien & Dürre, Nucleic Acids Isolation Methods, American Scientific Publishers, 2002).

In den bekannten Verfahren zur Präparation von Nukleinsäuren wird häufig Ethanol als Lösungsmittel verwendet. Generell wird heute z.B. als Waschpufferadditiv Ethanol eingesetzt (z.B. DE-A 198 56 064, US 2005/0079535).

Ethanol ist aber von der IATA (international Air Transportation Association) als gefährliches Material (HAZMAT, hazardous material) eingestuft. Dadurch werden beim Transport durch Flugzeuge zusätzliche Gebühren und Steuern fällig, Zudem sind im Anschluss an den Waschvorgang dann aufwändige Schritte zur Entfernung von Ethanol notwendig, da geringe Mengen von Ethanol nachfolgende Reaktionen wie die PCR bereits stark stören oder völlig blockieren können.

Es gibt bereits Bestrebungen, in Verfahren zur Reinigung von Nukleinsäuren Ethanol durch andere Substanzen zu ersetzen.

So schlägt die frühere Anmeldung der Anmelderin EP 08163623.5 die Verwendung von Bindungsmediatoren im Adsorptionsschritt vor, die gemäß IATA-Richtlinien als ungefährlich gelten, Unter anderem ist auch Tetraethylenglykol als Bindungsmediator genannt.

Die weitere frühere Anmeldung der Anmelderin EP 08163624 schlägt Waschpuffer vor, die weniger als 24 Vol,-%, besonders bevorzugt kein Ethanol enthalten, Als Lösungsmittel kommen verschiedene Lösungsmittel in Frage, unter anderem kann der Waschpuffer auch Tetraethylenglykol enthalten.

DE-A 102 53 351 weist darauf hin, dass die Verwendung von Alkohol oder Aceton in Verfahren zur Reinigung von Nukleinsäuren große Nachteile aufweist, So beeinträchtigen selbst Spuren von Alkohol die nachgeschalteten Reaktionen ganz erheblich, In üblichen Reinigungsverfahren für Nukleinsäuren ist daher immer ein Ethanolentfernungsschritt umfasst, Zur Vermeidung dieser Problematik schlägt das Dokument vor, den Einsatz alkoholischer Komponenten ganz zu vermeiden. Offenbart wird unter anderem ein alkoholfreier Waschpuffer, der NaCl, MgCl₂ sowie Tris/HCl enthält, Tetraethylenglykol ist nicht genannt.

WO 2004/042058 beschreibt Pufferformulierungen zur Isolierung, Reinigung und Rückgewinnung von kurzkettigen Nukleinsäuren, die monovalente und multivalente Kationen enthält, Auch hier wird ein alkoholfreier Waschpuffer offenbart, der NaCl, MgCl₂ und Tris/HCl enthält.

WO 02/44400 offenbart als Reagens zur Extraktion von Nukleinsäuren eine wässerige Lösung, die Natriummetasilikat und einen substituierten Ether enthält. Waschpuffer sind nicht erwähnt.

US 5,610,287 beschreibt alkoholfreie Waschpuffer, die Tris/HCl, NaCl sowie Tween 20 umfassen.

WO 2008/071384 offenbart die Verwendung von Tetraethylenglykoldimethylether in verschiedenen Schritten einer DNA-Päparation und -Reinigung.

Alle diese Vorschläge haben aber noch nicht zu einer in allen Aspekten befriedigenden Ersatzlösung für Ethanol geführt, Insbesondere ist bei allen beschriebenen Ersatzlösungsmittein nicht geklärt, wie sich die Anwesenheit der Waschpuffer-Bestandteile auf die nachfolgende DNA-Amplifikation auswirkt, Üblicherweise beeinträchtigen nämlich selbst kleine Mengen Lösungsmittel die Effizienz der PCR ganz erheblich.

Die Aufgabe der Erfindung besteht daher darin, eine Verbesserung des Präparationsschritts bereitzustellen, insbesondere bei der Präparation von DNA, wobei die nachfolgend durchgeführte PCR durch das verwendete Lösungsmittel nicht beeinträchtigt wird.

### Offenbarung der Erfindung

Überraschenderweise hat sich gezeigt, dass diese Aufgabe durch die Verwendung von Tetraethylenglykol gelöst werden kann und dass die Anwesenheit vom Tetraethylenglykol die im Anschluss an die DNA-Präparation durchgeführten Reaktionsschritte nicht beeinträchtigt.

Die Erfindung stellt daher Verfahren zur Amplifikation von DNA bereit, bei dem die Reaktionslösung Tetraethylenglykol enthält.

Bevorzugt stammt dabei das Tetraethylenglykol in der Reaktionslösung aus dem Eluat der DNA-Präparation an festen Oberflächen, das zur Präparation der DNA verwendet wurde.

Weiterhin betrifft die Erfindung ein Verfahren zur Präparation und Amplifikation von DNA, umfassend folgende Schritte:
a) Immobilisieren von in einer Lösung enthaltener DNA an einer Oberfläche
b) Waschen der Oberfläche mit einem Waschpuffer
c) Rückgewinnung der DNA durch Elution,
d) Durchführung einer DNA-Amplifikation mit dem in Schritt c) erhaltenen Eluat, **dadurch gekennzeichnet, dass** der Waschpuffer Tetraethylenglykol enthält.

Bevorzugt beträgt dabei die Menge an Tetraethylenglykol im Waschpuffer mindestens 10 Vol.-%, bevorzugt mindestens 48 Vol.%, noch bevorzugter mindestens 60 Vol.-%.

Ebenfalls bevorzugt beträgt in Schritt d) das Volumen des Eluats mindestens 10 Vol,%, bevorzugt mindestens 30 Vol,%, noch bevorzugter mindestens 50 Vol.-%, bezogen auf das gesamte Reaktionsvolumen der Amplifikationsreaktion.

Weiterhin bevorzugt ist eine Ausgestaltung des Verfahrens, bei der der Gehalt an Tetraethylenglykol im gesamten Reaktionsvolumen der Amplifikation bis zu 20 Vol.-%, bevorzugt bis zu 12 Vol,-%, noch bevorzugter bis zu 10 Vol.-% beträgt.

Der Waschpuffer kann darüber hinaus einen oder mehrere Bestandteile enthalten, die ausgewählt werden aus der chaotrope Salze und Puffersubstanzen enthaltenden Gruppe.

Die DNA-Amplifikation kann als Endpunkt-PCR, quantitative PCR oder isothermale Amplifikation durchgeführt werden.

Zudem stellt die Erfindung ein Verfahren nach einem oder mehreren der vorstehenden Ausgestaltungen bereit, das in einer mikrofluidischen Vorrichtung (Lab-on-a-Chip, LoC) durchgeführt wird.

Ein weiterer Aspekt der Erfindung ist ein Kit-of-parts zur DNA-Amplifikation und DNA-Präparation nach dem Verfahren nach einer oder mehreren der vorstehenden Ausgestaltungen , umfassend
- eine Lösung 1 als Waschpuffer, die Tetraethylenglykol enthält und
- für die Durchführung der DNA-Amplifikation notwendige Reagenzien.

Bevorzugt umfasst das Kit-of-parts darüber hinaus einen oder mehrere der folgenden Bestandteile, ausgewählt aus
- einer Lösung 2, umfassend einen Bindungsmediator,
- einer Lösung 3, umfassend ein Elutionsmittel
- einer Lösung 4, die einen Lysepuffer und ggf, eine oder mehrere Proteasen umfasst;
- einer mikrofluidischen Vorrichtung (Lab-on-a-Chip, LoC).

Die Erfindung betrifft weiter die Verwendung des vorstehenden Kit-of-parts für die Präparation und Amplifikation von aus biologischen Materialien gewonnener DNA, bevorzugt von aus für molekulardiagnostische Zwecke entnommene Proben (Human- und Veterinärmedizin), insbesondere aus forensischen Proben und aus in biologischen Proben wie z.B. Blut enthaltenen Viren oder Bakterien.

Schließlich betrifft die Erfindung die Verwendung von Tetraethylenglykol in einer Reaktionslösung zur Durchführung einer DNA-Amplifikation,

insbesondere einer Endpunkt-PCR, einer quantitativen PCR oder einer isothermalen Amplifikation, sowie eine Reaktionslösung zur Durchführung einer DNA-Amplifikation, umfassend für die Amplifikation notwendige Reagenzien und Tetraethylenglykol, wobei der Anteil an Tetraethylenglykol in der Reaktionslösung bevorzugt bis zu 20 Vol.-%, bevorzugt bis zu 12 Vol,-%, noch bevorzugter bis zu 10 Vol.-% beträgt.

### Beschreibung der Figuren:

Fig. 1 zeigt das Ergebnis der in Beispiel 1 durchgeführten PCR, die mit genomischer DNA aus E, coli O157:H7 in Vollblut bzw. PBS mit Ethanol als Waschpufferzusatz durchgeführt wurde.
Fig, 2 zeigt das Ergebnis der PCR mit genomischer DNA aus E, coli O157:H7 in PBS mit Ethanol bzw, Tetraethylenglykol als Waschpufferzusatz.
Fig. 3 ist eine Fotografie eines Agarose-Gels, die das Ergebnis einer Endpunkt-PCR von gereinigter humaner gDNA in Gegenwart steigender Tetraethylenglykol-Konzentrationen im Reaktionsansatz zeigt.
Fig. 4 und 5 zeigt das Ergebnis von Amplifikationen von RNA aus dem Bakteriophagen fr in Blut, die in Gegenwart von Ethanol bzw, Tetraethylenglykol durchgeführt wurden.
Fig. 6 zeigt das Ergebnis einer qPCR mit gereinigter RNA aus dem Bakteriophagen fr bzw, mit gereinigter humaner gDNA in Gegenwart steigender Konzentrationen Tetraethylenglykol im Reaktionsansatz.

DNA bedeutet erfindungsgemäß genomische DNA, gDNA, cDNA, Plasmid-DNA oder künstlich hergestellte DNA wie z.B, kurze DNA-Fragmente, Sie kann aus beliebigen Zellen oder Viren gewonnen oder künstlich hergestellt sein, Bevorzugt hat die DNA eine Länge von 1 2 oder mehr Basenpaaren, Sie wird z.B. aus prokaryotischen oder eukaryotischen Zellen wie Eubakterien (z.B. gram-positiven oder gram-negativen Bakterien), Archaeen , pathogenen Bakterien, eukaryotischen Mikroorganismen oder Zellen eines multizellulären Organismus gewonnen, z.B, Blutzellen, Gewebezellen usw, Sie kann aus einem natürlichen, künstlich modifizierten oder künstlich erzeugten Virus stammen, z.B. einem virulenten, abgeschwächten oder nicht-infektiösen Virus. Sie kann auch aus Pilzen, Hefen, Parasiten, Protozoen oder anderen Mikroorganismen stammen.

Die Mikroorganismen bzw, Zellen, aus denen die DNA erfindungsgemäß gewonnen wird, können aus beliebigen Proben erhalten werden wie z.B. Proben von Menschen, Tieren, Pflanzen oder Bodenproben; z.B. Blut, Urin, Gewebeproben, Haare, Speichel, Stuhl, Serum, Hirnflüssigkeit, forensischen Proben, Bodenproben, Pflanzenteilen wie Stängel, Blätter, Samen, Blüten oder Wurzeln.

Besonders vorteilhaft ist das erfindungsgemäße Verfahren für die Amplifikation von DNA aus in sehr geringen Mengen in der Probe vorliegenden Zellen, wie z.B. die DNA aus in Vollblut in geringen Titern vorliegenden Viren oder DNA aus forensischen Proben.

Es hat sich herausgestellt, dass das erfindungsgemäße Verfahren für die Präparation von RNA nicht geeignet ist. Der Grund liegt vermutlich darin, dass die für die Umschreibung der eluierten RNA in DNA notwendige Reverse Transkriptase erheblich empfindlicher gegen Tetraethylenglykol ist als gegen das üblicherweise als Waschpufferadditiv verwendete Ethanol.

In ihrer allgemeinsten Ausgestaltung betrifft die Erfindung die Durchführung einer DNA-Amplifikation, insbesondere durch PCR oder isothermale Amplifikation, wobei in der Reaktionslösung Tetraethylenglykol enthalten ist. Dieses stammt bevorzugt aus dem Waschpuffer, der in der vorgeschalteten Präparation der DNA verwendet wurde, d,h, aus dem Eluat, das nach der Präparation der DNA erhalten wird, Es kann aber auch erst später zur Reaktionslösung zugesetzt werden. Es hat sich gezeigt, dass selbst größere Mengen Tetraethylenglykol die PCR nicht inhibieren, was sehr überraschend ist angesichts der Tatsache, dass das üblicherweise verwendete Ethanol in sehr geringen Mengen bereits zu erheblichen Störungen der PCR führt und dass, wie Versuche der Anmelderin gezeigt haben, auch geringe Mengen Triethylenglykol (4 Vol.-% der Reaktionslösung für die PCR) bereits zu einer vollständigen Inhibierung der PCR führen.

Erfindungsgemäß kann die Reaktionslösung, die zur Durchführung der DNA-Amplifikation eingesetzt wird, bis zu 20 Vol.-% Tetraethylenglykol, bevorzugter bis zu 12 Vol.-% und noch bevorzugter bis zu 10 Vol,-% Tetraethylenglykol enthalten. Es hat sich gezeigt, dass auch bei Anwesenheit so großer Mengen Tetraethylenglykol keine Inhibierung der PCR stattfindet.

Prinzipiell kann die Präparation der DNA auf jede beliebige bekannte Art und Weise erfolgen.

Bevorzugt geht man erfindungsgemäß wie folgt vor:

Zunächst erfolgt die Lyse der Zellen, d,h, der Aufschluss und die Freisetzung der darin enthaltenen DNA in bekannter Weise durch Erhitzen, Ultraschall oder Mahlen mittels Beads, z.B. Glaskügelchen, Die Lyse erfolgt in einem üblichen Lysepuffer, Der Lysepuffer ist z.B. PufferAL (Fa. QIAGEN GmbH; Hilden, Germany), Weitere Lysepuffer sind Fachleuten bekannt und werden passend für den zu lysierenden Mikroorganismus ausgewählt, ebenso wie die geeigneten Lysebedingungen. Es können auch mehrere verschiedene Lysepuffer eingesetzt werden. Z, B, kann als Lysepuffer PBS-Puffer (phosphatgepufferte Kochsalzlösung) verwendet werden, der Proteinase K und Lysozym enthält. Gegebenfalls wird zunächst inkubiert und dann ggf, ein weiterer Puffer zugegeben wie z.B. Puffer G (GITC
(Guanidiniumthiocyanat)/Nonidet® (Nonylphenylethylenglykol)) und nochmals inkubiert. Das geeignete Volumen des/der Puffer muss in Abhängigkeit zum Probenmaterial bestimmt werden und beträgt z.B. je nach Probenmaterial zwischen 50 µl und 1 ml. Die Temperatur und Zeit zur Inkubation wird jeweils geeignet ausgewählt, Da manche Lyseverfahren mittels Einsatz von Enzymen erfolgen, ist die gewählte Temperatur auch abhängig von der Aktivität der Enzyme, Zymolase, Lyticase und Lysozym werden in der Regel im Temperaturbereich von 20°C - 37°C eingesetzt. Die Inkubation mit Proteasen , z.B. Proteinase K oder Subtilisin, erfolgt in der Regel bei 50 - 60°C. Die Dauer der Inkubation beträgt in der Regel 5 bis 20 Minuten, bevorzugt ca. 10 Minuten. Die Inkubation kann z.B. in einem Thermomixer durch Schütteln erfolgen, z.B. bei ca. 1400 Upm, oder auch auf einem Vortex®-Gerät (maximale Leistungsstufe)), ggf, unter Zusatz von Glasperlen.

Im Anschluss an die Lyse erfolgt die Isolierung der durch die Lyse freigesetzten Nukleinsäuren durch Immobilisierung (Adsorption) an festen Oberflächen.

Bei den Reinigungsverfahren durch Adsorption nutzt man das selektive Binden der Bestandteile des Inhalts des Lysats an oder auf einem festen Stütz- oder Trägermaterial ("Binden", Immobilisieren, Schritt a)), das Beseitigen unerwünschter Bestandteile vom festen Stütz- oder Trägermaterial ("Waschen", Schritt b)), und das Lösen des gewünschten Bestandteils ("Elution" Schritt c)), Dieses Vorgehen wird auch als "bind-wash-elute" bezeichnet.

Die Oberflächen, an denen die DNA erfindungsgemäß immobilisiert wird, sind übliche im Stand der Technik bekannte Oberflächen wie Membranen, Platten oder Beads. Sie bestehen aus Kunststoffmaterial wie z.B, Polystyrol, mineralischem Material wie Glaspulver, Diatomeenerden, Silicagelen, magnetischen Partikeln, Glasfaservliesen, Membranfiltern, Metalloxiden, Latexpartikeln, Am häufigsten werden Partikel auf Silica-Basis eingesetzt, Besonders vorteilhaft können im MagAttract®-Kit (Qiagen GmbH, Hilden, Deutschland) bereitgestellte magnetische Silica-Partikel eingesetzt werden, Die DNA wird so immobilisiert, dass sie durch Waschen mit Wasser nicht in nennenswerten Mengen vom Träger gelöst wird.

Üblicherweise erfolgt nach der Immobilisierung ein Waschschritt mit einem Waschpuffer, um unerwünschte, ebenfalls an der Oberfläche gebundene Bestandteile des Lysats zu entfernen.

Waschpuffer sind i.a. wässrige Lösungen. Erfindungsgemäß enthalten die Waschpuffer bevorzugt Tetraethylenglykol. Bevorzugt beträgt der Anteil an Tetraethylenglykol im Waschpuffer mindestens 10 Vol.-%, noch bevorzugter mindestens 48 Vol,-%, am bevorzugtesten mindestens 60 Vol,-%. Der Waschpuffer umfasst darüber hinaus übliche für Waschpuffer verwendete Substanzen, insbesondere (gereinigtes) Wasser, Salze wie z.B. Guanidiniumhydrochlorid, Natriumchlorid, und Puffer wie Tris/HCl, Besonders bevorzugt kann in den bekannten Waschpuffern AW1 und AW2 der Fa, Qiagen Ethanol durch Tetraethylenglykol ersetzt werden, so dass besonders bevorzugte erfindungsgemäße Waschpuffer TEG1 (48 Vol,-% Tetraethylenglykol, 2 M Guanidiniumhydrochlorid) und TEG2 (60 Vol,-% Tetraethylenglykol, 100 mM NoCl, 10 mM Tris/HCl pH 7,5) sind.

Der pH wird bevorzugt mit üblichen Puffersubstanzen auf einen gewünschten Wert eingestellt. Puffersubstanzen sind z.B. Tris/HCl, Natriumacetat, Maleat, Natriumcitrat. Der pH des erfindungsgemäß verwendeten Waschpuffers beträgt bevorzugt 7 bis 9.

Als chaotrope Salze, die dem Waschpuffer zugesetzt werden können, kommen erfindungsgemäß insbesondere Bariumsalze, Thiocyanate, Perchlorate, Guanidiniumsalze und insbesondere Natriumchlorid, Guanidiniumhydrochlorid, Natriumiodid, Natriumperchlorat und Guanidiniumthiocyanat in Frage.

Nach dem Waschen kann ggf, nach Bedarf ein Trocknen durchgeführt werden (z.B, Trocknen im offenen Gefäß an Luft, "air dry"), oder es kann mit Wasser gewaschen werden (water rinse), Dabei wird Wasser über die Festphasenpartikel geleitet, ohne diese dabei zu homogenisieren, Dieser Vorgang verringert die Menge an vorhandenem Bindungsmediator (z.B. Ethanol), ohne die gebundene Nukleinsäure von der Festphase zu lösen, Im Allgemeinen ist dies aber nicht notwendig, wenn Tetraethylenglykol im Waschpuffer verwendet wird, da dieses die nachfolgenden Reaktionen nicht stört. Lediglich für den Fall, dass der Waschpuffer kein Tetraethylenglykol, sondern Ethanol enthält, d.h. Tetraethylenglykol erst der PCR-Reaktionsmischung zugesetzt wird, ist das Trocknen und/oder Waschen mit Wasser erforderlich, um Ethanol zu entfernen.

Um die Nukleinsäure wieder von der Oberfläche zu lösen, wird anschließend eluiert, Dies geschieht regelmäßig mit einer schwach salzhaltigen ggf, gepufferten wässrigen pH-stabilisierten Lösung. Geeignete Elutionslösungen sind dem Fachmann bekannt.

Die so erhaltene DNA-Präparation wird dann amplifiziert, z.B. durch PCR oder isothermale Amplifikation.

Erfindungsgemäß besonders bevorzugt ist die Präparation von DNA mit der sogenannten MagAttract®-Technologie, bei der die DNA an magnetischen Silicapartikeln (MagBeads) adsorbiert wird. Diese Partikel sind von Qiagen erhältlich, Der Grund liegt darin, dass bei Verwendung dieser Partikel Substanzen aus dem Waschpuffer wie z.B. Ethanol oder Tetraethylenglykol nur schwer entfernt werden können. Durch Trocknen (air dry) gelingt dies nur bedingt, durch Waschen etwas besser. Beide Vorgehensweisen bedeuten jedoch aufwendige Zusatzschritte im Reinigungsprotokoll, Restliches, im Eluat vorhandenes Ethanol stört die nachfolgende PCR aber in erheblichem Maß bis zum Totalversagen, während Tetraethylenglykol sie überraschenderweise nicht stört.

Die PCR-Technik ist z.B. umfassend in PCR Technology: Principles and Applications for DNA Amplification, Erlich, Hrsg. (1992); PCR Protocols: A Guide to Methods and Applications, Innis et al" Hrsg, (1990), R.K, Saiki et al., Science 230 ;1350 (1985) und US 4,683,202 beschrieben.

Die PCR ist eine enzymatische Methode zur Synthese spezifischer Nukleinsäuresequenzen unter Verwendung von zwei Oligonukleotid-Primern (Sonden), die mit entgegengesetzten Strängen hybridisieren und einen interessierenden Abschnitt einer Target-Nukleinsäure flankieren, Eine Serie von wiederholten Reaktionsschritten, welche Templat-Denaturierung, Primer-Annealing und Verlängerung der hybridisierten Primer durch die DNA-Polymerase umfassen, resultiert in exponentieller Akkumulation des spezifischen Target-Fragments, dessen Enden durch die 5'-Enden der Primer definiert sind. Das PCR-Verfahren verwendet wiederholte Zyklen der DNA-Synthese zur Replikation der Target-Nukleinsäure, In der grundlegenden Ausführungsform umfasst die PCR folgende Schritte:
- Zugabe spezifischer Primer zu einer Probe, von der vermutet wird, dass sie die Target-Nukleinsäure enthält. Die Primer sind so gewählt, dass sie an komplementäre DNA-Stränge binden, die in entgegengesetzten Strängen der DNA vorliegen. Die Target-Nukleinsäure ist innerhalb der Primerbindungsstellen lokalisiert und ist ein Marker des nachzuweisenden Organismus,
- Zugabe der vier Desoxynukleosid-Triphosphate, Magnesiumsulfathaltiger Puffer, Polymerase-Enzyme und von verschiedenen Additiven und Cosolventien, Mischen mit der Probe und den Primern
- Denaturieren der Probe durch Erwärmen, Trennung der DNA in zwei komplementäre Stränge
- Abkühlung, dadurch Bindung (Hybridisierung) der Primer an die jeweiligen Bindungsstellen an den komplementären DNA-Strängen
- Primer-Extension auf dem DNA-Templat durch DNA-Polymerase.

Diese drei Schritte Denaturierung, Hybridisierung und Extension werden 40 bis 50 Mal wiederholt und resultieren in einer exponentiellen Amplifikation der Targetsequenzen.

Die PCR in Schritt d) kann erfindungsgemäß als Endpunkt-PCR oder als Realtime-PCR (quantitative PCR, qPCR) durchgeführt werden. Die Vorgehensweise ist dem Fachmann bekannt. EP-A-0 512 34, EP-A-0 640 828, EP-A-0 519 338 (F, Hoffmann- La Roche AG) offenbaren beispielsweise die qPCR, Es werden hierfür kommerziell Systeme angeboten, z.B. TaqMan® (Roche Molecular Systems, Inc., Branchburg Township, New Jersey).

Für die Durchführung der PCR sind erfindungsgemäß alle für die DNA-PCR gängigen Reagenzien und dafür verfügbaren Kits einsetzbar. Die Primer, Desoxynukleotid-Triphosphate, Polymerasen, die Additive, Cosolventien und Markierungs-Sonden sind, wie eingangs beschrieben, dem Fachmann bekannt und werden geeignet ausgewählt, Beispielsweise können für eine TaqMan®-Analyse entsprechende PCR-Reagenziensätze von QIAGEN, wie der QuantiTect® Probe PCR Mastermix bzw. der QuantiFast® Probe RT-PCR MasterMix verwendet werden, Z,B, ist QuantiTect® PCR-Mastermix, QuantiTect®5x Virus-Mastermix oder HotStarTaq®-Mastermix erfindungsgemäß für die Durchführung der PCR geeignet.

Die Primer werden geeignet ausgewählt und können mit Fachleuten bekannten Verfahren synthetisch hergestellt werden oder sind kommerziell z.B. bei den Firmen Operon oder MWG Biotech erhältlich.

Als Marker können alle Marker verwendet werden, die Fachleuten bekannt sind. Insbesondere werden zur Detektion Fluoreszenzmarker verwendet, z.B. 6-FAM, TET, Quasar® und/oder HEX, Der Fachmann wird je nach Bedarf auch andere Fluorophore einsetzen.

Als Lösungsmittel für die PCR-Reagenzien und Primer wird üblicherweise Wasser verwendet.

Die Reaktionslösung für die Amplifikation enthält erfindungsgemäß Tetraethylenglykol, bevorzugt in einer Menge von bis zu 20 Vol,-%, bevorzugt bis zu 12 Vol,-%, noch bevorzugter bis zu 10 Vol.-% bezogen auf die Reaktionslösung, Es hat sich überraschenderweise gezeigt, dass auch in Anwesenheit derartig großer Mengen Tetraethylenglykol die PCR sehr zuverlässig verläuft und erfolgreich durchgeführt werden kann.

Für die Amplifikation von RNA ist dagegen eine Reaktionslösung, die bereits geringe Mengen Tetraethylenglykol enthält (z.B. ab 1 Vol,-%), ungeeignet, da die Amplifikationseffizienz sich in diesem Fall extrem verschlechtert. Bei einem Gehalt von 9 Vol,-% TEG im Reaktionsansatz ist bei der RNA-Amplifikation meist gar keine Reaktion mehr zu beobachten.

Die Temperaturzyklen der PCR werden vom Fachmann je nach verwendeter PCR-Chemie, zugrundeliegender Ausgangs-DNA sowie gewählter Primer geeignet ausgewählt und können z.B. 15 Min bei 95°C und 40 x [15 Sekunden bei 95°C, 1 Minute bei 60°C] sein, Die Kontrolle der Temperatur und das Erwärmen und Abkühlen erfolgen auf übliche Weise.

Die Amplifikation kann erfindungsgemäß auch als isothermale Amplifikation durchgeführt werden, Isothermale Amplifikationsverfahren sind im Stand der Technik bekannt. Sie haben gemeinsam, dass keine Temperaturzyklen angewendet werden, sondern dass kontinuierlich eine bestimmte Reaktionstemperatur beibehalten wird. Beispiele für derartige Verfahren, die für die Amplifikation von DNA geeignet sind und daher erfindungsgemäß zur Anwendung kommen können, sind HDA (Helicase Dependent Amplification, siehe M. Vincent et al., EMBO reports 2004, 795-800), RPA (Recombinase Polymerase Amplification, siehe O, Piepenburg et al., PloS Biology, 2006, 1115-1120), EXPAR (exponential amplification reaction, siehe z.B. J, Van Ness et al., PNAS 2002, 4504-4509].

Es kann auch eine Hugl-PCR durchgeführt werden. Hugl ist ein Target in humaner genomischer DNA. Das für diese PCR verwendete Primerpaar wurde vor längerer Zeit von Qiagen entwickelt. Die damit durchgeführte PCR ist sehr empfindlich gegen Störungen und wird daher dazu genutzt, um zu zeigen, dass durch DNA hergestellte Präparate besonders sauber sind bzw. kaum bzw. keine inhibitorischen Verunreinigungen enthalten.

Der Nachweis der Amplifizierungsprodukte kann je nach den verwendeten Markern wie eingangs beschrieben auf unterschiedliche Weise erfolgen, Dies ist dem Fachmann bekannt. Bei Verwendung von Fluoreszenzmarkern erfolgt die Detektion fluoreszenzspektrometrisch, In üblichen PCR-Verfahren können bis zu 6 verschiedene Fluorophore gleichzeitig nachgewiesen werden (6-Kanal-Multiplexing). Es wird dann die Anzahl von PCR-Zyklen bestimmt, ab der ein deutlicher Anstieg der Fluoreszenz des spezifischen Markers auftritt (Ct, threshold cycle).

Die klassische PCR-Reaktion ist eine Endpunkt-PCR. Dadurch ist es möglich, die Anwesenheit oder Abwesenheit einer Target-DNA nachzuweisen, die Methode gibt aber keine Auskunft über die Anfangskonzentration der Target-DNA.

In neuerer Zeit wurde die Real-Time PCR (RT-PCR; Echtzeit-Polymerase-Chain-Reaktion; auch quantitative PCR (qPCR) genannt) entwickelt. Diese ermöglicht das gleichzeitige Verfolgen von mehreren Nukleinsäure-Amplifikationsreaktionen durch PCR, wobei die akkumulierten Daten zur quantitativen Bestimmung der Ausgangskonzentration einer Target-Nukleinsäuresequenz verwendet werden. Daher wird diese PCR-Raktion auch als Multiplex-PCR bezeichnet. Offenbart ist die Real-Time PCR z.B. in EP-A-0 512 34, EP-A-0 640 828, EP-A-0 519 338 (F. Hoffmann- La Roche AG). Es werden hierfür kommerziell Systeme angeboten, z.B. TaqMan® (Roche Molecular Systems, Inc., Branchburg Township, New Jersey). Das Verfahren ermöglicht die schnelle und präzise Quantifizierung der Ausgangskopienzahl über einen sehr weiten Konzentrationsbereich. Es ist geeignet für viele Anwendungen wie Genexpressionsstudien, Sequenz- und Mutationsanalysen, Überprüfung von Virustitern, den Nachweis pathogener oder genetisch modifizierter Organismen. Ein Kit für Real-Time PCR mit sequenzspezifischen Sonden ist z.B. erhältlich von Eppendorf AG, Hamburg (RealMasterMix Probe^{®}) oder von Qiagen GmbH, Hamburg (artus^{®} PCR Kits),

Bei der TaqMan^{®} PCR sind die Oligonukleotidsonden so ausgelegt, dass sie an der Target-Nukleinsäuresequenz zwischen den Extensionprimern binden. Jede Oligonukleotidsonde ist am 5'-Ende mit einem Reportermolekül wie einem Fluorophor und am 3'-Ende mit einem Reportermolekül-Quencher markiert. Die markierten Sonden werden gemeinsam mit den Primern und der Probe zur PCR-Reaktionsmischung gegeben. Nach der Denaturierung wird die Reaktionsmischung abgekühlt, wobei die markierten Sonden bevorzugt an die Target-Nukleinsäuresequenz binden. Als nächstes wird bis auf die optimale Temperatur zur Primerbindung und Extension abgekühlt. Im Verlauf der Polymerisation, wenn die DNA-Polymerase entlang dem Target-Nukleinsäurestrang vom 3'-Ende zum 5'-Ende fortschreitet, wobei Nukleotide an den wachsenden Primer angefügt werden, trifft der Primer auf die 5'-Enden der zuvor an den Target-Nukleinsäurestrang gebundenen markierten Sonden, Wenn die DNA-Polymerase auf diese markierten Sonden trifft, übt sie ihre 5'-3'-Exonucleaseaktivität aus und baut die markierten Sonden ab. Fluorophore und Quencher werden dabei in die Reaktionsmischung freigesetzt. Der TaqMan® Assay ist so ausgelegt, dass Reportermoleküle, die innerhalb einer vorherbestimmten Nähe des Quenchermoleküls vorliegen, nahezu keine Fluoreszenz aussenden. Daher wird in der PCR-Mischung zu Beginn keine Fluoreszenz aufgrund des Reportermoleküls nachgewiesen, da die Fluoreszenz des Reportermoleküls vom in räumlicher Nähe befindlichen Quenchermolekül gelöscht wird, Während der PCR werden die 5'-fluoreszenzmarkierten Sonden freigesetzt und dabei von den 3'-Fluoreszenzquenchern getrennt. Nach der Freisetzung wird der Fluoreszenzmarker nicht mehr gequencht und kann daher durch Fluoreszenzspektrometrie oder durch andere geeignete Mittel nachgewiesen werden. In der Reaktionsmischung vorhandene ungebundene Sonden stören nicht, da sie gequencht bleiben. Unspezifisch an Nukleinsäuresequenzen, die nichts mit der Target-Nukleinsäure zu tun haben, gebundene markierte Sonden stören ebenfalls nicht, da sie gebunden und daher gequencht bleiben. Daher ist jedes freie Reportermolekül, das in der Reaktionsmischung nachgewiesen wird, direkt proportional zu der Menge der ursprünglich spezifisch gebundenen markierten Sonde, und daher der Target-Nukleinsäure, Zum Nachweis unterschiedlicher Target-Nukleinsäuren in einer Probe werden Sonden mit unterschiedlichen Fluoreszenz-Reportermolekülen verwendet, die in unterschiedlichen Wellenlängenbereichen fluoreszieren und daher den gleichzeitigen Nachweis verschiedener Target-DNAs ermöglichen. Als Fluoreszenz-Reporter sind z.B. 6-FAM, VIC, Bodipy-TMR, JOE und HEX bekannt und geeignet, als Quencher TAMRA, MBG, Black Hole Quencher 1 (BHQ1), Black Hole Quencher 2 (BHQ2), Black Hole Quencher 3 (BHQ3), BodyPi 564/570 und Cy5 oder ähnliche, Der Fachmann wählt die Reporter und Quencher geeignet aus, bevorzugt so, dass eine gegenseitige Überlagerung der Fluoreszenzemissionen ("Bleed through") vermieden wird. Z.B. ist die Kombination 6-FAM/BHQ1 besonders geeignet.

Neben Fluoreszenzmarkern können auch radioaktive Marker, chemilumineszente Marker, paramagnetische Marker, Enzyme und Enzymsubstrate als Marker verwendet werden.

Im Anschluss an die Amplifikation erfolgt die Detektion der Nukleinsäuren durch Fluoreszenzspektrometrie, wie vorstehend beschrieben, oder bei Verwendung anderer Marker durch entsprechende andere Detektionsverfahren.

Die genannten Nukleinsdurepräparations- und -amplifikations- sowie detektionsverfahren können im Labormaßstab, d.h. im Mikro- oder Millilitermaßstab durchgeführt werden, Alternativ wurden in jüngerer Zeit mikrofluidische Verfahren entwickelt. Auch diese sind erfindungsgemäß einsetzbar.

Unter Mikrofluidik wird die Handhabung und das Arbeiten mit kleinen Flüssigkeitsmengen verstanden, die ein um Zehnerpotenzen geringeres Volumen aufweisen als normale Flüssigkeitstropfen.

Vorrichtungen zur Durchführung mikrofluidischer Verfahren, die eine Probe bis zum gewünschten Messergebnis verarbeiten, werden auch als Lab-on-a-Chip (LoC) bezeichnet, also Einweg-Verbrauchsmaterial als Labor auf einem Chip in der Größe etwa einer Kreditkarte, mit dem molekularbiologische Bestimmungen vorgenommen werden, z.B. die Bestimmung von Erregern einer Infektion, Nahrungsmittelkontrolle, Veterinärdiagnostik, Analyse biologischer Kampfstoffe, Umweltanalytik, Für die Analyse ist nur geringer Personalaufwand und keine spezielle Ausbildung oder Einarbeitung zur Bedienung erforderlich, LoC werden in ein Betreiberinstrument von der Größe eines Videorecorders eingelegt, das dann das Ergebnis ausgibt, Ein LoC vereinigt mikrofluidische Funktionen zur Probenextraktion und Anreicherung des Analyten, zur Signalverstärkung (Amplifikation) und Detektion in einem Chip. Die Bedienung des LoC und der Bedieneinheit ist sehr einfach, und die Analyse erfolgt extrem schnell verglichen mit konventionellen Analyseverfahren (30-60 Minuten Gesamtprozesszeit gegenüber mindestens 6 Stunden).

### Beispiele:

Escherichia coli O157:H7 ist bei der American Type Culture Collection unter Nr, ATCC 700728 erhältlich.

Gereinigte humane gDNA wurde mit Hilfe des QIAamp® DNA Midi Kits aus humanem Blut hergestellt.

Bakteriophage fr ist erhältlich bei der American Type Culture Collection unter Nr, ATCC 15767-B1.

### Abkürzungen die im Folgenden benutzt werden:

- QA =: QIAamp®, erhältlich bei Qiagen
- MasG =: MagAttract® Suspension G, erhältlich bei Qiagen
- PBS =: phosphate buffered saline, Phosphat-gepufferte Kochsalzlösung
- AW =: QIAGEN Waschpuffer AW (1/2), erhältlich bei Qiagen
- TEG =: Tetraethylenglykol
- SPE =: Festphasenextraktion von Nukleinsäuren (mittels QA o. MasG)
- qPCR =: quantitative PCR
- RT-qPCR =: Reverse Transkription -quantitative PCR
- BHQ =: Block Hole Quencher

In den nachfolgenden Beispielen werden die Auswirkungen verschiedener Waschprozeduren auf die Effizienz der Endpunkt- bzw. Realtime-PCR beschrieben. Das dabei beschriebene Waschen mit TEG wurde jeweils mit den Puffern TEG1 sowie TEG2 als Substitut für AW1 und AW2 durchgeführt. Die Puffer TEG1 und TEG2 setzen sich wie folgt zusammen:
TEG1: 48 Vol.-% Tetraethylenglykol, 2.5M Guanidinium-HCl, Rest Wasser
TEG2: 60 Vol.-%% Tetraethylenglykol, 100mM NaCl, 10mM Tris/HCl pH 7,5, Rest Wasser

### Beispiel 1:

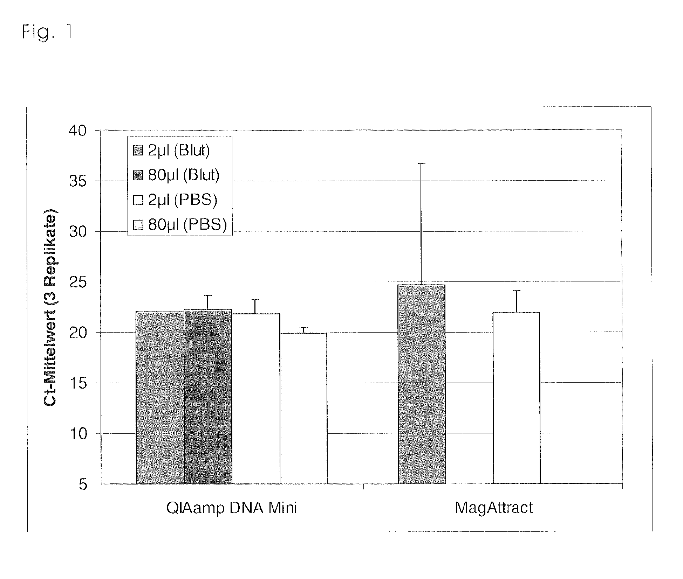

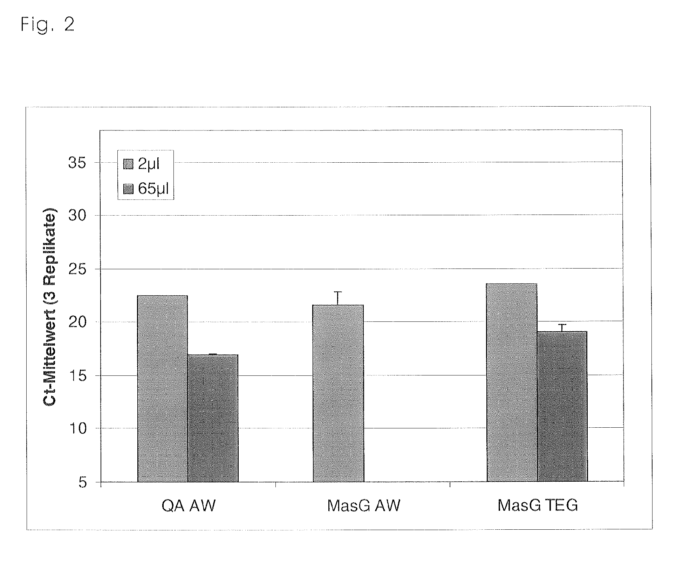

40µl einer Übernachtkultur von E. coli O157:H7 wurde in 800 µl Vollblut bzw, PBS gegeben. Dazu wurden 80 µl QIAGEN Proteinase K sowie 800 µl Puffer AL gegeben und gemischt. Dieses Gemisch wurde nun 15 bei 56 °C inkubiert. Das so hergestellte Lysat wurde für alle weiteren Aufreinigungen der enthaltenen DNA genutzt. Die DNA-Reinigung erfolgte je 3x mit Hilfe des QIAamp® DNA Mini Kits bzw, mittels eines manuelle MagAttract® Protokolls, Die Reinigung mittels QA DNA Mini Kit® erfolgte entsprechend den Vorgaben des Herstellers und umfassten 2 Waschschritte mit AW1 bzw, AW2, eine kurze Trocknung der gebundenen Nukleinsäure durch Luft und Elution mit deionisiertem Wasser. Das MasG® Protokoll umfasste folgende Schritte: 200µl Lysat wurden mit 200 µl Isopropanol und 20 µl MasG® durch kurzes Vortexen gemischt und anschließend 5 min mit 1400 Upm auf einem Thermomixer geschüttelt. Danach wurden die MasG® Beads magnetisch separiert und der Überstand abgenommen und verworfen. Die MasG® Beads wurden durch iteratives Resuspendieren im entsprechenden Puffer, nachfolgendes magnetisches Separieren und Abnehmen des Überstands je 4x mit Waschpuffer AW1 bzw. AW2 gewaschen. Nachfolgend wurden die separierten Beads 15min im offenen 1,5m1-Reaktionsgefäß an Luft getrocknet und danach in 100µl Wasser resuspendiert. Zur Elution der DNA wurden die im Wasser verteilten Beads 2 min geschüttelt. Abschließend wurden die Beads separiert und der die DNA enthaltende Überstand abgenommen. Die so erhaltenen DNA-Lösungen wurden in eine qPCR Reaktion eingesetzt. Dies erfolgte durch Vorlegen von 40 µl QuantiTect® PCR-Mastermix (Qiagen GmbH), 40 pmol Forward-Primer (SEQ ID NO:1; 5'-CGATGATGCTACCCCTGAAAAACT-3'), 40 pmol Reverse-Primer (SEQ ID NO:2; 5'-TATTGTCGCTTGAACTGATTTCCTC-3'), 20pmol Sondenoligo (SEQ ID NO:3; FAM-CGTTGTTAAGTCAATGGAAAACCTG-BHQ1) in einer 96er PCR-Platte und Eintrocknen dieses Gemisches über Nacht bei 37°C. Der eingetrocknete Reaktionmix wurde mit 80 µl Eluat bzw, 78 µl Wasser plus 2 µl Eluat rehydratisiert und 5 min bei 1400 Upm auf einem Thermomixer geschüttelt, Danach wurde das rückgelöste Reaktionsgemisch in einem Real Time Cycler (MJ Research Opticon, Biorad) folgendem Temperaturverlauf unterworfen: 15 min 95 °C, 40x [15 sec 95°C, 1 min 60 °C]. Das Ergebnis ist in Fig. 1 dargestellt.

Ergebnis: Während die QA-Präparation den Einsatz großer Mengen an Eluat für die nachfolgende Präparation erlaubt, ist dies für Eluate, die mit der MasG®-Technologie gewonnen werden, nicht möglich. Ein möglicher Grund hierfür ist die unvollständige Ethanol-Entfernung von den MasG®-Beads vor der DNA-Elution.

### Beispiel 2:

Wie in Beispiel 1 beschrieben, wurde ein gemeinsames Lysat aus einem Gemisch von 100 µl E, coli O157:H7, 3 ml PBS, 300 µl Qiagen Proteinase K sowie 3 ml GIN3 [3M Guanidiniumisothiocyanat, 20% NP-40 (Nonidet P-40, Octylphenoxylpolyethoxyethanol)], Dieses gemeinsame Lysat wurde, mit folgenden Ausnahmen, exakt wie in Beispiel 1 beschrieben durchgeführt, Zum Waschen der gebundenen Nukleinsäure wurden neben AW1 / AW2 auch TEG1 / TEG2 benutzt. Bei der MasG®-basierten Aufreinigung wurde je 2x mit Waschpuffer 1 und 2 gewaschen, Bei den mit TEG-basierten Waschpuffern behandelten Proben wurden die DNA-behafteten MasG® Beads statt durch 1 5-minütige Trocknung mit 500 µl Wasser gespült, Bei diesem Spülen wurden die Beads nicht resuspendiert, sondern das Reaktionsgefäß verblieb während des Spülvorgangs in der magnetischen Halterung, die sicherstellt, dass die Beads als kompakter Haufen von der Flüssigkeit separiert bleiben. Dieser Schritt ist notwendig, da TEG auf Grund seines sehr hohen Siedepunkts bei Raumtemperatur, im Gegensatz zu Ethanol, praktisch nicht verdampft. Die so erhaltenen DNA-Lösungen wurden in eine qPCR Reaktion eingesetzt. Dies erfolgte exakt wie in Beispiel 1 beschrieben durch Rehydratisieren eines vorher getrockneten Reaktionsmixes mit 65 µl Eluat bzw, 63 µl Wasser plus 2 µl Eluat. Die PCR Bedingungen sind ebenfalls mit denen in Beispiel 1 identisch, Das Ergebnis ist in Fig, 2 dargestellt,

Ergebnis: Sollen große Mengen Eluat aus einer MasG®-DNA-Präparation in einer nachgeschalteten qPCR eingesetzt werden, so führt die Verwendung von Ethanol-haltigen Waschpuffern (AW1/2) zu Inhibitionseffekten, die einen Totalausfall der PCR verursachen. Werden stattdessen TEG-haltige Waschpuffer verwendet (TEG1/2), kann ein zuvor getrockneter PCR-Mastermix ausschließlich mit diesem Eluat rekonstituiert und erfolgreich eine qPCR durchgeführt werden.

### Beispiel 3:

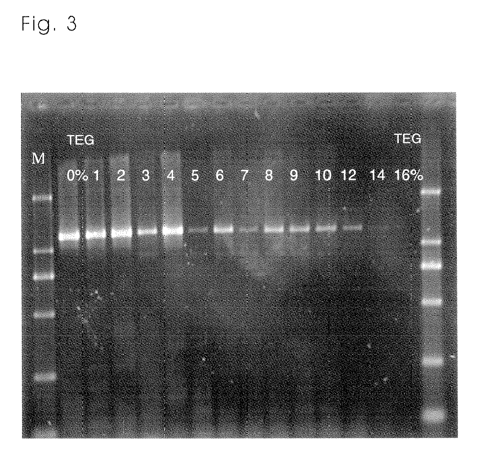

Es wurde eine PCR im Tumor-Supressorgen Hugl (Abk, für human giant larvae) von 3 ng gereinigter humaner gDNA mit dem HotStarTaq®-Mastermix (25µl Endvolumen; 10pmol Forward-Primer (SEQ ID NO:4; 5'-CACACAGCGATGGCAGCTATGC-3'], 10pmol Reverse-Primer [SEQ ID NO:5; 5'- CCCAGTGATGGGCCAGCTC-3') in Gegenwart steigender Konzentrationen TEG im Reaktionsansatz mit dem Temperaturprofil 15 min 95°C, 40x [30 sec 95°C, 30 sec 59 °C, 1 min 72 °C], 5 min 72 °C durchgeführt. Es ist bekannt, dass diese Reaktion mit den verwendeten Primern sehr störanfällig ist, z.B. gegenüber Verunreinigungen des DNA-Templates, Die Ausbeute an PCR-Produkt in den Reaktionsansätzen wurden abschließend durch Agarosegel-Elektrophorese begutachtet. Das Ergebnis ist in Fig. 3 dargestellt.

Ergebnis: Die hier durchgeführte Hugl-PCR lässt sich auch in Anwesenheit großer Mengen TEG (10 Vol-%) erfolgreich durchführen.

Die Beispiele 1-3 demonstrieren die Überlegenheit von TEG-haltigen gegenüber Ethanol-haltigen Waschpuffern beim Einsatz der MagAttract®-Technologie zur Reinigung von DNA. TEG-Waschpuffer sind insbesondere vorteilhaft, wenn große Mengen des hergestellten DNA-haltigen Eluats in enzymabhängigen Amplifikationsreaktionen (hier quantitative PCR) eingesetzt werden sollen, Diese Option ist besonders wichtig, wenn das verwendete Probenmaterial nur geringste Nukleinsäuren-Mengen enthält (Viren in geringen Titern, einzelne Zellen in forensischem Material etc).

Die nachfolgenden Beispiele demonstrieren, dass TEG-haltige Waschpuffer ungeeignet sind zur RNA-Extraktion aus biologischen Proben, Da TEG die PCR-Reaktion, wie oben gezeigt, nicht negativ beeinflusst, ist diese Beobachtung vermutlich auf eine Störung der Aktivität der Reversen Transkriptase durch TEG zurückzuführen,

### Beispiel 4:

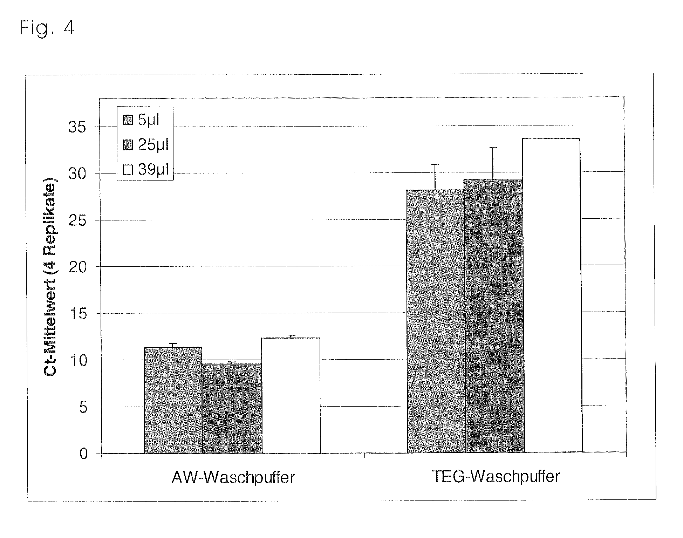

Ein gemeinsames Lysat aus einem Gemisch von 30 µl Bakteriophage fr (ein Bakterien spezifischer Virus mit RNA basiertem Genom), 3 ml Blut, 300 µl Qiagen Proteinase K sowie 3 ml GIN3 wurde 15 min bei 56°C inkubiert. Die SPE (solid phase extraction, Festphasenextraktion) erfolgte ausschließlich per MagAttract® Verfahren. Der große Lysatansatz wurde, ähnlich wie in Beispiel 1 beschrieben, in 600 µl Portionen aufgeteilt und mit jeweils 300 µl Isopropanol sowie 30 µl MasG® versetzt. Zum Waschen der gebundenen Nukleinsäure wurden wiederum entweder AW1 / AW2 oder TEG1 / TEG2 benutzt, Es wurde 1x mit 1ml AW1 bzw, TEG1 sowie 2x mit 0,5ml AW2 bzw, TEG2 gewaschen. Daran schloss sich eine 5-minütige Lufttrocknung an. Final wurde die gereinigte RNA mit 100 µl Wasser eluiert, Alle SPE-Schritte (Binden/Waschen/Eluieren) wurden mit Hilfe des Robotors BioSprint® 15 (Qiagen, Hilden) durchgeführt, Die so erhaltenen RNA wurde nachfolgend in einer RT-qPCR eingesetzt. Dazu wurden 5 µl, 25 µl bzw. 39 µl Eluat mit 1 0 µl1 QuantiTect® 5x Virus-Mastermix (Qiagen, Hilden), je 20 pmol Primern (Forward: SEQ ID NO:6; 5'- CTTCTGATCCGCATAGTGACGAC -3'; Reverse: SEQ ID NO:7; 5'- AACGGTCATTCGCCTCCAGCAG - -3') und 1 0 pmol Sonde (SEQ ID NO:8; 6-FAM- TAGGGGATGGTAACGACGAAGCA-BHQ1)gegen das fr-Genom sowie 0,5 µl QuantiTect® Virus RT Mix gemischt (Endvolumen 50 µl), Folgendes Temperaturprofil diente der cDNA Herstellung und Amplifikation: 20 min 50 °C, 5 min 95°C, 40x [15 sec 95 °C, 45 sec 60°C], Das Ergebnis ist in Fig. 4 gezeigt,

Ergebnis: Grundsätzlich zeigen die mit AW-Puffern gewaschenen Eluate eine deutlich bessere RTqPCR-Performance (kleinerer Ct-Wert) als die mit TEG-Puffern gewaschenen Ansätze. Weiterhin zeigt sich, dass der Einsatz großer Mengen TEG-Eluat (39µl auf 50µl RTqPCR-Ansatz) nicht nur zu einem schlechteren Ct-Wert gegenüber dem Einsatz kleiner TEG-Eluat-Mengen (5µl auf 50µl Endvolumen) führt, sondern auch zu einem Totalausfall der Amplifikationsreaktion führen kann (lediglich 1 von 4 Ansätzen mit 39µl TEG-Eluat pro 50µl Reaktionsansatz zeigte eine erfolgreiche Amplifikationsreaktion).

Eine Erklärung für die schlechte Performance der Tetraethylenglykolgewaschenen Proben ist die nicht gewährleistete Abreicherung von TEG aufgrund der durchgeführten Lufttrocknung. TEG weist eine Siedetemperatur von 306 °C aufweist und wird somit bei einer Lufttrocknung praktisch nicht in die Gasphase übergehen.

### Beispiel 5:

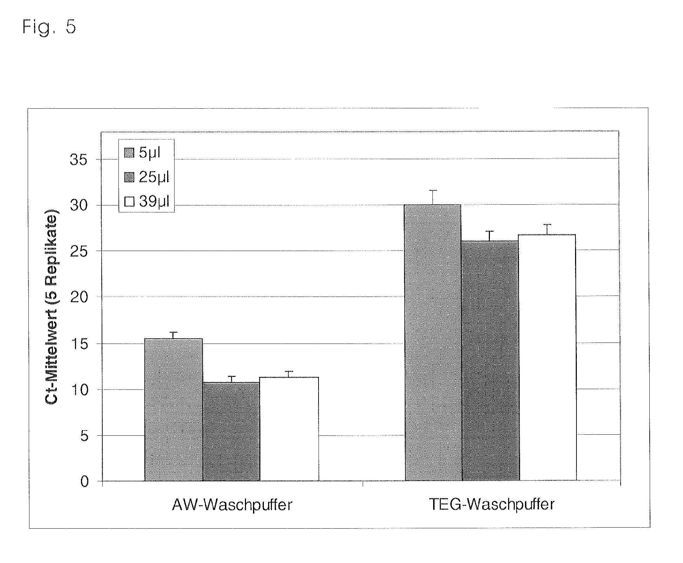

Auch in diesem Beispiel wurde Bakteriophage fr in humanes Blut gegeben und lysiert sowie die RNA gereinigt. Alle Lyse- und SPE-Schritte wurden identisch zu Beispiel 4 durchgeführt. Um nach den Waschschritten verbliebenes TEG möglichst effektiv zu entfernen wurde diesmal jedoch die Lufttrocknung durch eine Spülung mit Wasser (water rinse) ersetzt. Für die nachfolgenden RT-qPCRs wurden wiederum 5 µl, 25 µl bzw, 39 µl Eluat mit den gleichen PCR-Komponenten wie in Beispiel 4 zu einem Endvolumen von 50 µl gemischt. Fig. 5 zeigt das Ergebnis,

Ergebnis: Wie in Beispiel 4 zeigen die mit AW-Puffern gewaschenen Eluate eine deutlich bessere RTqPCR-Performance als die mit TEG-Puffern gewaschenen Ansätze. Diesmal fällt die Inhibition bei Einsatz großer Mengen TEG-Eluats (39 µl auf 50 µl RTqPCR-Ansatz) wesentlich geringer aus, Dies zeigt, dass das Waschen mit Wasser restliches TEG besser von den MagBeads® entfernt als ein Trocknen an Luft, Trotzdem blieb bis dahin nicht erklärlich, warum die TEG-Eluate grundsätzlich schlechtere RTqPCR Ergebnisse erzielten als die AW-Eluate,

### Beispiel 6:

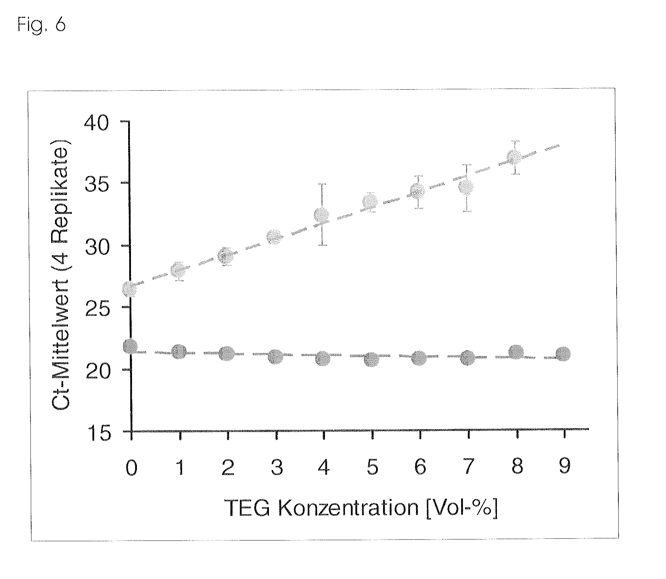

Der in Beispiel 5 verbliebenen Frage, warum TEG-Eluate grundsätzlich schlechtere RTqPCR-Ergebnisse liefern als AW-Eluate, wurde in folgendem Experiment nachgegangen. Eine RTqPCR (QuantiTect® Probe RTPCR Mastermix) von gereinigter RNA aus Bakteriophage fr bzw, eine qPCR (QuantiTect® Probe PCR Mastermix) von gereinigter humaner gDNA (Forward-Primer: SEQ ID NO:9; 5'-GCCGCTAGAGGTGAAATTCTTG-3', Reverse-Primer: SEQ ID NO:10; 5'-CATTCTTGGCAAATGCTTTCG-3', Sonde: SEQ ID NO:11; HEX-ACCGGCGCAAGACGGACCAGA-BHQ1) wurde in Gegenwart steigender Konzentrationen an TEG im Reaktionsansatz durchgeführt (je 4 Replikate). Das verwendete Temperaturprofil war 30 min 50 °C, 15 min 95 °C, 4x [15 sec 95 °C, 1 min 60°C], Das Ergebnis ist in Fig. 6 dargestellt.

Ergebnis: TEG verschlechtert auch in größeren Konzentrationen nicht die Performance der PCR-Reaktion bei DNA, Dieses Ergebnis bestätigt die in Beispiel 3 vorgestellten Daten der Hugl-PCR, Demgegenüber führen jedoch bereits geringe Mengen TEG (ab 1 Vol-%) zu einer dramatischen Verschlechterung der RTqPCR-Reaktionseffizienz bei RNA, Bei 9 Vol-% TEG im Ansatz war keine der 4 RTqPCR-Reaktionen erfolgreich (keine Amplifikation erkennbar).

## Patentansprüche

1. Verfahren zur Amplifikation von DNA, **dadurch gekennzeichnet, dass** die Reaktionslösung Tetraethylenglykol enthält,

2. Verfahren nach Anspruch 1 , wobei das Tetraethylenglykol in der Reaktionslösung aus dem Eluat der DNA-Präparation an festen Oberflächen stammt, das zur Präparation der DNA verwendet wurde.

3. Verfahren zur Präparation und Amplifikation von DNA, umfassend folgende Schritte:
a) Immobilisieren von in einer Lösung enthaltener DNA an einer Oberfläche
b) Waschen der Oberfläche mit einem Waschpuffer
c) Rückgewinnung der DNA durch Elution,
d) Durchführung einer DNA-Amplifikation mit dem in Schritt c) erhaltenen Eluat,
**dadurch gekennzeichnet, dass** der Waschpuffer Tetraethylenglykol enthält.

4. Verfahren nach Anspruch 3, wobei die Menge an Tetraethylenglykol im Waschpuffer mindestens 10 Vol,-%, bevorzugt mindestens 48 Vol,%, noch bevorzugter mindestens 60 Vol.-% beträgt.

5. Verfahren nach Anspruch 3 oder 4, wobei in Schritt d) das Volumen des Eluats mindestens 10 Vol.%, bevorzugt mindestens 30 Vol,%, noch bevorzugter mindestens 50 Vol.-% beträgt, bezogen auf das gesamte Reaktionsvolumen der Amplifikationsreaktion.

6. Verfahren nach einem der Ansprüche 1 bis 5, wobei der Gehalt an Tetraethylenglykol im gesamten Reaktionsvolumen der Amplifikation bis zu 20 Vol,-%, bevorzugt bis zu 1 2 Vol.-%, noch bevorzugter bis zu 1 0 Vol,-% beträgt.

7. Verfahren nach einem oder mehreren der Ansprüche 3 bis 6, wobei der Waschpuffer darüber hinaus einen oder mehrere Bestandteile enthält, die ausgewählt werden aus der chaotrope Salze und Puffersubstanzen enthaltenden Gruppe.

8. Verfahren nach einem oder mehreren der Ansprüche 1 bis 7, wobei die DNA-Amplifikation als Endpunkt-PCR, quantitative PCR oder isothermale Amplifikation durchgeführt wird.

9. Verfahren nach einem oder mehreren der Ansprüche 1 bis 8, **dadurch gekennzeichnet, dass** es in einer mikrofluidischen Vorrichtung (Lab-on-a-Chip, LoC) durchgeführt wird.

10. Kit-of-parts zur DNA-Amplifikation und DNA-Präparation nach dem Verfahren nach einem oder mehreren der Ansprüche 3 bis 9, umfassend
- eine Lösung 1 als Waschpuffer, die Tetraethylenglykol enthält und
- für die Durchführung der DNA-Amplifikation notwendige Reagenzien,

11. Kit-of-parts nach Anspruch 1 0, darüber hinaus umfassend einen oder mehrere der folgenden Bestandteile, ausgewählt aus
- einer Lösung 2, umfassend einen Bindungsmediator,
- einer Lösung 3, umfassend ein Elutionsmittel
- einer Lösung 4, die einen Lysepuffer und ggf, eine oder mehrere Proteasen umfasst;
- einer mikrofluidischen Vorrichtung (Lab-on-a-Chip, LoC).

12. Verwendung eines Kit-of-parts nach einem der Ansprüche 10 und 1 1 für die Präparation und Amplifikation von aus biologischen Materialien gewonnener DNA.

13. Verwendung eines Kit-of-parts nach einem der Ansprüche 1 0 bis 1 2 für die Präparation und Amplifikation von DNA aus für molekulardiagnostische Zwecke entnommene Proben (Human- und Veterinämedizin), insbesondere aus forensischen Proben und aus in Blut enthaltenen Viren oder Bakterien.

14. Verwendung von Tetraethylenglykol in einer Reaktionslösung zur Durchführung einer DNA-Amplifikation.

15. Verwendung nach Anspruch 14, wobei die DNA-Amplifikation eine Endpunkt-PCR, quantitative PCR oder isothermale Amplifikation ist.

16. Reaktionslösung zur Durchführung einer DNA-Amplifikation, umfassend für die Amplifikation notwendige Reagenzien und Tetraethylenglykol.

17. Reaktionslösung nach Anspruch 13, wobei der Anteil an Tetraethylenglykol in der Reaktionslösung bis zu 20 Vol,-%, bevorzugt bis zu 1 2 Vol.-%, noch bevorzugter bis zu 1 0 Vol.-% beträgt,
